# EUROPEAN PATENT SPECIFICATION

(11) **EP 1 808 129 B1**
(45) Date of publication and mention of the grant of the patent: **03.05.2017**
(21) Application number: 05788145.0
(22) Date of filing: 26.09.2005
(51) Int. Cl.: A61B 6/14, A61B 6/03, A61B 6/00

(54) **CROSS SECTION INFORMATION DETECTION DEVICE**
VORRICHTUNG FÜR QUERSCHNITTSINFORMATIONEN
DISPOSITIF DE DETECTION D'INFORMATIONS EN COUPE TRANSVERSALE

(30) Priority: 24.09.2004 JP 2004308871; 09.03.2005 JP 2005111375; 09.03.2005 JP 2005111374
(43) Date of publication of application: 18.07.2007
(73) Proprietor: iCAT CORPORATION, Osaka 532-0004 (JP)
(72) Inventor: SOGO, Motofumi, c/o iCAT Corporation, Yodogawa-ku, Osaka-shi, Osaka 5320004 (JP); SOMEKAWA, Yoshihisa, c/o iCAT Corporation, Yodogawa-ku, Osaka-shi, Osaka 5320004 (JP); NAKAI, Ryusuke, c/o iCAT Corporation, Yodogawa-ku, Osaka-shi, Osaka 5320004 (JP)
(74) Representative: D Young & Co LLP
(86) International application number: PCT/JP2005/018221
(87) International publication number: WO 2006/033483

(56) References cited:
- EP-A1- 1 207 495
- WO-A1-00/41132
- WO-A1-03/037204
- WO-A2-03/037189
- JP-A- 1 125 681
- JP-A- 2 088 046
- JP-A- 2003 245 289
- JP-A- 2003 260 056
- JP-A- 2005 253 727
- US-A- 5 921 927
- US-A1- 2002 015 006
- US-B1- 6 243 439

## Description

### BACKGROUND OF THE INVENTION

### Field of the Invention

There is described a device that extracts and processes human body information relative to a reference plane of a human body element from imaged information, which is imaged not by accurately positioning the human body element, such as CT, and a method for converting the imaged information, such as CT, into imaged information relative to a reference plane of the human body element. The present invention relates to a cross-section information detector that detects cross-section information of a human body element including an optionally selected reference axis, from the CT image information of the human body element.

### Description of the Related Art

CT imaging is available as one method for detecting diseases in affected areas within patients. This CT imaging is an imaging method for acquiring tomographic images within patients; however, there is a defect that even though any tomography at the imaged site can be planarly and visually confirmed because of two-dimensional analog information, the information cannot be perceived as a three-dimensional image. In recent years, in order to resolve this defect, a system where the two-dimensional analog information acquired from the CT imaging is converted into three-dimensional digital information and the converted information is displayed on a display as a three-dimensional image has been developed. In this system, while an operator visually confirms the three-dimensional image at the site within the patient on the display, a tomographic image of the site within the patient can be acquired by specifying any position of said image. In actuality, in order to display the image of the three-dimensional information of the site within the patient acquired from the CT imaging on the display, the information of the site within the patient (hereafter, referred to as 'human body information') is arranged on a virtual space having absolute coordinates referred to as a world area and the image will be displayed in the interest of processing.

However, in the system, even though the operator can visually confirm the three-dimensional image on the display, image information viewed from a reference position desired by a dentist cannot be acquired. This is because the CT image information is merely arranged on the absolute coordinates on the world area, and because no imaging information relative to the reference position is specified. For example, explaining a case of CT imaging of the neck of a patient for implanting a dental implant (hereafter, referred to as an 'artificial dental root') into a portion missing from a tooth alignment, the CT is imaged when the head of the patient is placed on the bed, and the imaged tomography information will be cross-section information position-based upon the bed.

In the meantime, the tomography information required by a dentist is information where a human body element desired by the dentist is regarded as positional reference according to a treatment mode. Therefore, a dentist (or an operator instructed by a dentist) who images the CT needs to image a patient by positioning based upon a desired positional reference. This is a very difficult task and requires experience; in the meantime, if the degree of exposure is considered, there are circumstances that re-imaging cannot be repeated. Because of these circumstances, a means that detects CT image information of a site within a patient positioned to any position as human body information including relative positional information from a reference plane (like an occlusal plane) of a site within the patient has been in demand among healthcare professionals.

Further, in the case of displaying an image of the three-dimensional information from the CT image information, human body information and other noise are often mixed and displayed, so it is actually difficult to visibly identify the desired human information. For example, there is the following problem: noise information referred to as an artifact generated from other elements added to the human body element, such as metal pieces, typically exists, and even if a dentist tries to visually confirm and understand the three-dimensional information of a site within a patient by the CT imaging, the noise information becomes an obstacle and desired site information cannot accurately be acquired.

In addition, in order to resolve the problems, various technologies have been developed and examined in recent years; in the meantime, other problems, such as slow processing speed or a jumbo-sized device, are also pointed out.

Particularly, it is desirable for a dentist who conducts an implant surgery to acquire cross-section information based upon an implant to be implanted from imaged information, such as CT; however, in the case of imaging, such as conventional CT, the dentist provides a treatment based upon coordinates of view where an occlusal plane is visually confirmed from the front side of a patient; in the meantime, the CT image information is displayed with the coordinate system where the world area is visually confirmed from the outside, so there is also another problem that a cross-sectional image, which is different from the view in a treatment stage, is provided to the dentist. This is a task requiring that a dentist needs a lot of experience to conduct a surgical operation by viewing the cross-sectional image. According to these circumstances, dental professionals have desired the provision of cross-section information of patients based upon any reference position, and in addition, cross-section images required from the treatment viewpoint by dentists.

For example, as the related art, Japanese Unexamined Patent Application Publication No. 2002-177262, Japanese Unexamined Patent Application Publication No. 2001-000430 and U.S. Pat. No. 6,704,439.
EP 1207495 A1 describes a three-dimensional image display.

### DISCLOSURE OF THE INVENTION

### Problem to be solved by the Invention

The present invention has been invented by taking these circumstances into consideration, and the objective is to provide a human body information extractor where three-dimensional information, which is not accurately positioned because of simple processing, can be positioned onto a desired reference plane, and in addition, where noise information can be eliminated. Further, the objective of the present invention is also to provide a conversion method for a reference plane of human body imaging information where three-dimensional image information of a patient, such as CT image information, is converted into three-dimensional image information by simple and reliable processing based upon a human body reference plane. In addition, the objective is also to provide a cross-section information extractor that detects cross-section information around a predetermined reference axis among human body information, and that further provides the cross-section information always from a desirable viewpoint of dentists.

### Problem Resolution Means

An aspect of the present invention are set out in the appended claim.

According to a human body information extractor, information of a desired portion in a human body element is extracted and detected from three-dimensional image information of said human body element positioned at any position within a space. Specifically, the human body information extractor comprises a human body information arrangement means that arranges the three-dimensional image information of the human body element in the state, where a positioning member to be positioned at a fixed position relative to a reference plane of the human body element is arranged, at any position within a world area; a reference plane detection means that detects positional information on the world area, where multiple specific portions of the positioning member occupy, respectively, from the three-dimensional information arranged within the world area, and that detects the positional information of the reference plane on the world area from said positional information; and a reference positional information detection means that detects positional information of the human body element in the world area relative to the reference plane.

Further, it is preferable that the specific portions of the positional member in the human body information extractor are arranged so as to project from the human body element when the positioning member is positioned to the human body element, and to position each center on the reference plane. The reference plane detection means can comprise a means that detects a relative positional relationship between an assigned point selected as any point on the surface of each specific portion and its vicinity point from the three-dimensional information of the human body element arranged within the world area; a means that detects the center position of the specific portion from the relative positional relationship between the detected assigned point and its vicinity point; and a means that detects a plane within the world area including the detected center positions of the specific portions as a reference plane. Further, it is preferable that this device comprises a reference area specifying means that specifies a desired area from the reference plane within the world area; and a reference area information extraction means that extracts human body information arranged within the area specified by the reference area specifying means.

Herein, for example, the three-dimensional image information of the human body element contains at least three-dimensional image information of the maxillary, the mandible and the positioning member, and the maxillary and the mandible include a tooth alignment, respectively. Further, it is preferable that the positioning member is positioned while being intervened between the tooth alignments of the maxillary and the mandible, and that the position where this positioning member is fixed is specified as a reference plane or an occlusal surface.

Further, the human body information extractor may comprise a model information arrangement means that arranges three-dimensional image information of a human body model prepared to have roughly the same external shape as a desired portion of the human body element as one unit of an object on the world area to be displaceable; a redundant information detection means that detects the human body information arranged within the area redundant to the three-dimensional image information of the human body model arranged by the model information arrangement means within the world area; and a means that extracts the human body information corresponding to the redundant portion detected by the redundant information detection means. In addition, the human body model is prepared based upon the human body element where the positioning member to be positioned at a fixed position relative to the reference plane of the human body element is arranged, and it is composed of at least a model of a human body element and a model of a positioning member. The human body information extractor comprises a model reference plane detection means that detects positional information on the world area, where multiple specific portions of a model of the positioning member equivalent to the multiple specific portions of the positioning member occupy, respectively, and that detects positional information of a reference plane of the human body model equivalent to the reference plane of the human body element from this positional information; and a human body model reference plane setting means that sets the reference plane detected by the model reference plane detection means to the three-dimensional image information of the human body model arranged by the model information arrangement means. At least when the positional information for the reference planes of the human body model is matched with that of the human body element, it is preferable that the redundant information detection means extracts the three-dimensional image information of the human body element arranged within the area redundant to the three-dimensional image information of the human body model. Furthermore, it is preferable that the human body model is prepared based upon the maxillary of the human body element, the mandible and the positioning member positioned by being intervened between the tooth alignments of the maxillary and the mandible.

Further, when the three-dimensional image information of the human body element is CT image information, it is normal that the CT image information is composed of spatial information of the human body element and its corresponding CT values, and in this case, it is preferable that [the human body information extractor] comprises a first specified CT value setting means that sets a CT value, which is unique to the multiple specific portions of the positioning member, as a first specified CT value; a second specified CT value setting means that sets a CT value, which is unique to the portion other than the human body information in the CT image information, as a second specified CT value; a first actually measured CT value detection means that detects the CT value at the specific portion in the CT image information as a first actually measured CT value; a second actually measured CT value detection means that detects the CT value of the portion other than the human body information in the CT image information as a second actually measured CT value; a function information setting means that sets function information of the actually measured CT value to the specified CT value from the first specified CT value and its corresponding first actually measured CT value, and the second specified CT value and its corresponding second actually measured CT value; and a CT value calibration means that calibrates the actually measured CT value in the CT image information to the specified CT value by comparing to the function information set by the function information setting means. Further, the human body information extractor may comprise a specified distance setting means that sets a specified distance between desired specific portions among the multiple specific portions of the positioning member; an actually measured distance measuring means that measures a distance between desired specific portions in the three-dimensional image information arranged within the world area; and a positional information calibration means that calibrates the three-dimensional image information so as to match the actually measured distance with the specified distance.

In addition, [the human body information extractor] comprises a tooth alignment information setting means that sets a dental arch where each dental crown image information is arranged at a tooth alignment position corresponding to each dental crown on the reference plane, and a dental crown image display means that displays a desired dental crown image on said reference plane, and that designates other dental crown images not to be displayed. In addition, on the occasion of imaging a human body model, the human body model is provided with a fixture for a three-dimensional imaging having an upper contact section and a lower contact section, which have a positional relationship at a predetermined distance in a vertical direction, and a connecting section that connects the upper contact section and the lower contact section. It is preferable that this upper contact section can maintain the contact with the maxilla of the human body model on the lower surface directly or via a predetermined member; concurrently, can separate them, and the lower contact section can maintain the contact state of the mandible of the human body model on the upper surface directly or via a predetermined member; concurrently, can separate them. In addition, the positional relationship between the upper contact section and the lower contact section is positioned to enable the maintenance of the insertion of the model of the positioning member between the maxilla and the mandible in the state where the maxilla and the mandible are maintained to come into contact with the both contact sections, respectively.

In addition, a conversion method for a reference plane of human body image information where three-dimensional imaging information of a human body element positioned at any position within a space into information where one human body reference place is considered as a reference position, comprises: a model reference plane detection step for matching one member in a fixture of a model with another human body reference plane when a model of a human body element in a patient is fixed to the fixture, and detecting one human reference plane with respect to one member of the fixture; a fixture image acquiring step for imaging the fixture where the model is fixed, and acquiring three-dimensional image information; a patient image acquiring step for imaging a human body element of the patient, and acquiring the three-dimensional image information; a patient image reference plane detection step for overlapping both three-dimensional imaging information by positioning one member of the fixture in the three-dimensional image information of the fixture acquired at the fixture image acquiring step to a position of another human body reference plane in the three-dimensional image information of the patient acquired at the patient image acquiring step, and detecting the three-dimensional image information of the patient positioned at one human body reference plane detected at the model reference plane detection step among three-dimensional image information of the fixture as one human body reference plane, and a step for converting the three-dimensional image information where the human body element of the patient is imaged into image information whereby the one human body reference plane detected at the patient image reference detecting step is considered as a reference position.

Further, when utilized in dentistry, this is a method for converting the three-dimensional image information in the vicinity of the jaw positioned at any position within a space into information whereby an occlusal plane is considered as a reference position, comprising: a model occlusal plane detection step for detecting an occlusal plane with respect to an upper member of the fixture by matching the upper member of the fixture with a camper plane on the model when the upper and lower tooth alignment models of a patient are fixed to the fixture; a fixture image acquiring step for imaging the fixture where the tooth alignment models are fixed, and acquiring three-dimensional image information; a patient image acquiring step for imaging a human body element of the patient, and acquiring its three-dimensional image information; a patient image reference plane step for overlapping both three-dimensional imaging information to the position of the camper plane on the three-dimensional image information of the patient acquired at the patient image acquiring process by positioning the upper member of the fixture on the three-dimensional image information acquired at the fixture image acquiring step, and detecting the three-dimensional image information of the patient positioned at the occlusal plane detected at the model reference plane detection step among the three-dimensional imaging information of the fixture; and a conversion step for converting the three-dimensional image information where the human body element of the patient is imaged into image information where the occlusal plane detected at the patient image reference plane detection step is regarded as a reference position. Herein, the case of matching the upper member of the fixture with the camper plane is described; however, an eye-ear plane, which is a reference plane used in dentistry other than the camper plane, may be used.

Further, as a tool to execute the method, a fixture equipped with an upper member and a lower member, which are opposing in parallel, and a connecting member whose one end is pivotally connected to be rotatable with regard to said upper member at said end, and whose other end is fixed to the lower member is provided. The upper member of this fixture is a member for detecting an upper occlusal plane when an upper tooth alignment model of the patient is fixed so as to be positioned on the camper plane in said upper alignment model, and the lower member is a member for detecting a lower occlusal plane in parallel to the lower member, when the connecting member is rotated so as to position the lower tooth alignment of the patient to have normal occlusion with the upper tooth alignment model, and the lower member is fixed. In addition, the upper member, the lower member and the connecting member of the fixture are made from a material with low X-ray transmissivity, respectively, or at least the upper and lower members are made from a material with low X-ray transmissivity, respectively. Even in this fixture, the upper member can be a member for detecting the upper occlusal plane, when the upper tooth alignment model of the patient is fixed so as to position the upper member on an eye-ear plane in said upper tooth alignment model.

In addition, a cross-section information detector in the present application detects cross-section information containing an optionally selected reference axis from three-dimensional image information of a human body element. This device comprises a means that produces a predetermined plane area including a reference axis; a means that specifies the reference axis and the plane area at predetermined positions of the human body element; and a human body information extraction means that extracts human body information positioned at the plane area where the reference axis and the plane area are specified. Further, it is preferable that this human body information extraction means comprises a means that specifies multiple points of detection in a minute area (such as voxel or pixel) within the plane area; a means that detects a plurality of human body information within the world area corresponding to each point of detection; a means that averages a plurality of the detected human body information, and that sets the information as one human body information in the minute area; and a means that detects one human body information in the set minute area throughout the plane area.

Further, according to this cross-section information detection means, the reference axis can be inclined in a predetermined direction based upon the human body element, and the plane area is inclined along with the reference axis, and in addition, the reference axis may be rotatable while the reference axis and the plane area are maintained to be inclined when the reference axis is inclined in said directional component. Further, the reference axis can be inclined in a predetermined direction based upon the human body element, and the plane area will not be inclined regardless of the inclination of the reference axis, and in addition, when being inclined in the directional component, the reference axis may be rotatable while the inclined state is maintained, and the plane area may be rotatable while the not inclined state is maintained.

In addition, the three-dimensional imaging information of the human body element in this cross-section information detector applies the one mainly composed of the maxilla and/or the mandible including a tooth alignment, respectively. It is preferable that the reference axis can be inclined in a tooth alignment direction; the plane area is inclined in the tooth alignment direction along with the reference axis; and when the reference axis is inclined in the tooth alignment direction, it is rotatable while the reference axis and the plane area are maintained to be inclined. Further, this reference axis can also be inclined in a buccolingual direction. In this case, it is preferable that the plane area is inclined in the buccolingual direction along with the reference axis; when being inclined in the buccolingual direction, the reference axis is rotatable while the inclined state in the buccolingual direction is maintained; and the plane area is rotatable while the non-inclined state in the buccolingual direction is maintained.

Further, the cross-section information detector may comprise a position detection point setting means that sets coordinates of two or more desired positional points of position detection to the plane area; and a positional relationship measuring means within the plane area that sets a distance between the points of detection using each coordinate of said point of detection when the number of the set points of position detection is two, and that measures an angle of line segments formed with any two points among said points of detection and/or a distance between said line segments using the coordinates of the points of detection.

In addition, the three-dimensional image information of the maxilla and the mandible is set by overlapping with the three-dimensional image information of the human body model formed only with a hollow or thin-wall surface at least having tooth alignments contained in said maxilla and mandible, and the human body information extraction means may extract the three-dimensional image information of the maxilla and mandible positioned in the plane area and the three-dimensional image information of the human body model positioned in said plane area.

### Efficacy

The human body information extractor can position three-dimensional information of a human body element obtained from CT image information where a patient cannot be precisely positioned on a display. Specifically, a positioning member is arranged on a reference plane, such as an occlusal plane, and CT is imaged, and images of information where the imaged information is three-dimensionally digitalized (or information where medical data is also included in said information: also generally referred to as "three-dimensional information of human body element" or "human body information") is displayed on a world area having world coordinates by the human body information arrangement means. On these displayed images, a positioning member fixed to each patient is also displayed in addition to the patient himself/herself, and an operator, such as a dentist, can visually confirm this on the display and detect a reference plane (such as an occlusal plane), which will be a positional reference, by designating a specific portion(s).

Further, the human body information extractor detects the reference plane (such as an occlusal plane) not only from the three-dimension information from the detection but also from the three-dimensional information of tooth alignment model from the CT image information, matches these on the display, and extracts the imaged information of the patient corresponding to the information from the tooth alignment model (hereafter, referred to as 'fitting'). With this function, imaged information of the patient, which is not accurately positioned but simply arranged on the world area, can be positioned in the local coordinate system relatively positioned based upon the reference plane, and in addition, human body information excluding noise information, which is normally generated at the time of CT imaging, can be extracted.

In addition, it is also possible to calibrate a difference from an initial CT value, which used to be executed on the occasion of CT imaging of a patient (there was a case of no calibration), after the CT imaging. Specifically, since information of the positioning member is contained in the CT image information, if a CT value for the positioning member whose specified CT value is recognized and another CT value for another portion(s) (such as a void portion) whose specified CT value is recognized are calibrated so as to match with the CT values obtained from the CT image information, even though the calibration is not executed before the CT imaging, it becomes possible to calibrate them after the event, so highly precise CT image information can be provided. Further, it becomes possible to calibrate a difference in the Z-axis direction (CT imaging direction), which has conventionally been pointed out in CT images), as well. In other words, the positioning member to be used for detecting a reference plane of a human body element can be simultaneously used as a calibration tool.

Further, it is also possible to extract only data within a desired range among the imaged information of the human body, such as CT, as another dual-purpose function of the positioning member. For example, a focused site regarding the occlusal plane detected by the positioning member can be extracted and the maxilla or mandible can automatically be separated. These functions can limit an extraction area to a desired range, and each processing thereafter can be drastically accelerated. Further, the human body information extractor can arrange a dental crown image in the vicinity of a portion missing from a tooth alignment within a patient. In addition, the fixture for imaging enables CT imaging in a state where the positioning member is inserted and maintained between the maxilla and the mandible of the human body model; concurrently, enables the CT imaging while the positional relationship between the maxilla and the mandible is maintained. With this function, only images of the maxilla and the mandible from the human body information extracted by the human body information extractor can be displayed; the imaging of the positioning member can be eliminated; and then, the operability by an operator who processes [the image] while visually confirming the image can be improved.

Further, with the conversion method for a reference plane of human body imaged information and the fixture, a CT image 300 of a patient can be considered as a CT image based upon the upper and lower occlusal planes, respectively. With this design, even if CT of a patient is imaged with any posture, the image information, which is easily understood by dentists based upon an occlusal plane, can be acquired. Further, even in a patient who cannot have a normal occlusion because a missing portion in a tooth alignment is [considerably great], the CT image information based upon the occlusal plane can easily be acquired, as well. Further, the fixture is for detecting an occlusal plane using a camper plane or an eye-ear plane, and it has a function similar to a well-known articulator (occlusal plane confirmation device) referred to as an average value articulator or an adjustment articulator from this viewpoint, so it is user-friendly for dentists who normally use this device. Because the fixture is made from a material with low X-ray transmissivity, such as acryl, the problem of artifact can also be avoided.

With the cross-section information detector, cross-section information around a reference axis desired by a dentist can be acquired. Further, because a medical member can also be displayed with the reference axis, the dentist can acquire the cross-section information in the coordinate system based upon the medical member while mentally imaging his/her own treatment situation on the display. For example, for a dentist who desires to define an implant position for an implant surgery, while a situation to implant a dental crown into a tooth alignment is visually confirmed, the implant position so as not to come into contact with the nerve, such as a mandibular canal, can be detected. In addition, in the cross-section information detector, the cross-section information can be acquired in the coordination of view by a dentist on the occasion of surgical operation.

In addition, with the cross-section information detector, vagueness in an image, which is unique to a CT image, can be eliminated, and usability by an operator who processes the implant image of an artificial dental root based upon the CT image and its cross-section information is drastically improved.

### BRIEF DESCRIPTION OF THE DRAWINGS

FIG. 1 shows a three-dimensional image of the calyx obtained from CT image information.
FIG. 2 is a flowchart showing steps for detecting an occlusal plane by the human body information extractor.
FIG. 3 is a flowchart showing other steps for detecting an occlusal plane by the human body information extractor.
FIG. 4 is a rough illustration showing a positioning member, and FIG. 4-1 and FIG. 4-2 are a side view and a view along a line X-X, respectively, and FIG. 4-3 and FIG. 4-4 are a side view and a view along a line X-X, respectively.
FIG. 5 is a schematic view showing a situation where the reference planes (occlusal planes) match with each other by the human body information extractor.
FIG. 6 shows situations to set the reference plane and a plane area on the display by the cross-section information detector.
FIG. 7 is a flowchart showing steps for detecting cross-section information around the reference axis by the cross-section information detector.
FIG. 8 are views showing a CT imaging fixture that fixes the upper and lower tooth alignment models, fitting using this fixture, and marking of the upper and lower tooth alignments.
FIG. 9 is a view showing a cross sectional image of the maxilla and the mandible.
FIG. 10 is an image view showing a positional relationship between the CT image of the maxilla and the mandible, the reference axis and the plane area on the occasion of acquiring a cross sectional image by the cross-section information detector.
FIG. 11 (a) shows a method for fixing a tooth alignment to the fixture used in the conversion method for a reference plane of human body imaged information, and FIG. 11 (b) shows a state where said fixture pivots.
FIG. 12 shows a technique for detecting a reference plane (occlusal plane) by overlapping the fixture onto the imaged information of a patient (CT image) in the conversion method for a reference plane of human body imaged information.
FIG. 13 shows modification examples of the conversion method for a reference plane of human body imaged information in FIG. 11 and FIG. 12.

### BEST MODE FOR IMPLEMENTING THE INVENTION

A CT image is a tomographic image of the calyx in a patient, and it is two-dimensional analog information. Therefore, in order to visually confirm this information as three-dimensional information, after the two-dimensional analog information is digitalized, the digitalized information is converted into three-dimensional information, and an image of the patient's calyx will be displayed on a display based upon the converted three-dimensional information. Further, the three-dimensional information obtained from the CT image information is detected information of a composition state of human body element in a patient, and it is merely composed basically with positional information (displacement information) as it is. However, if it is three-dimensionally digitalized, an image can be displayed as three-dimensional human body information containing medical information, such as other clinical data belonging to dentists. Therefore, if designating any point or any plane on the display, a dentist can visually confirm a tomographic image or medical information of the corresponding portion.

However, the three-dimensionally displayed image of the three-dimensional information is processed for composition to be an image based upon the corresponding positional information and/or medical information per unit area (voxel) of the image, and a tissue image (such as the calyx in a patient) is displayed as an assembly group of each voxcel display. Specifically, a three-dimensional spatial axis where an image is displayable is set as a world area, which is a spatial axis on the image, for image processing, and image display information corresponding to information per voxel is formed in one coordinate system (world coordinate system) to determine a position of the world area, and if the image is displayed throughout the entire world area and the image per voxel is regarded as an assembly group, it is visually confirmed as the tissue image of the calyx within the patient. The three-dimensional image of the calyx displayed on the display is merely an ensemble of separately displayed points, respectively, and it is not formed from the information of the calyx or each site or tissue to compose this [image]. In addition, the CT image information is information imaged by positioning (placing) a patient onto a bed, and because this is information where the patient information in any coordinates (equivalent to the world coordinates) from a fixed point of the CT imaging equipment (such as a bed) is acquired, even though the positional information of the calyx, which is an imaging subject, with regard to the CT imaging equipment can be displayed, no information about which position it is positioned is included, and in the case of displaying the image on the display, the calyx displaying state (such as an angle of inclination of the neck or an angle of swing) cannot be detected. In other words, even if the human body information within the coordinates specified on the world coordinates can be detected from the three-dimensional image based upon the CT image, this cannot be considered as relative information using an occlusal plane or a face-ear plane as a reference plane by a dentist.

Therefore, it is required to convert the three-dimensional information of the calyx positioned at any position within the world area to a position relative to a desired reference position, and to compose the calyx information formed according to the positional relationship based upon this reference position. Therefore, the present embodiment, first, provides a device that detects a reference plane, which will be an assumption to position the three-dimensional human body information that has not been positioned from the medical viewpoint at the time of CT imaging; in other words, that detects a reference plane for positioning from the human body information in the world coordinate system on a computer. Specifically, in the human body information extractor in the present embodiment, an occlusal plane, which is highly usable from a dentistry viewpoint, is adopted as a reference plane, and it is designed to detect positional information of this occlusal plane in the world coordinate system.

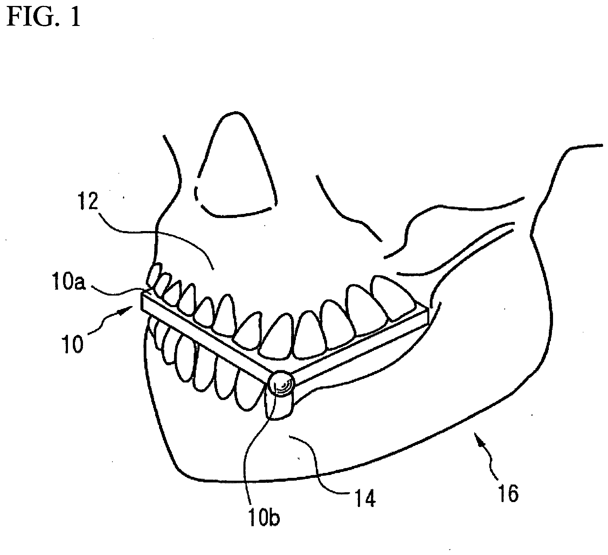

It is presumed that a patient wears the positioning member at the time of CT imaging in the human body information extractor of the present embodiment. Specifically, a case of using [this device] for dental treatment is described as an example. With reference to FIG. 1, this is a perspective view of the calyx 16 showing that a positioning member 10 in the human body information extractor is engaged with an occlusal plane in a patient (to be easily viewed, only the vicinity of upper and lower tooth alignments are shown, and in actuality, other calyx elements will be displayed). As it is understood from the view, the positioning member 10 is composed of a plate member 10a and three globular bodies 10b. When the patient occludes, the plate member 10a comes into contact with an upper tooth alignment 12 and a lower tooth alignment 14 both onto the upper and lower planes, respectively, and although the contacted plate member is generally flat, it may have flexibility to some extent of widely fitting onto the upper and lower tooth alignments 12 and 14. Therefore, the plate member 10a of the positioning member 10 is arranged along the occlusal plane.

If the patient is CT-imaged in this situation, an image of the calyx 16 displayed on the display also becomes a three-dimensional image of the calyx 16 where the positioning member 10 is mounted as similarly to FIG. 1. Furthermore, in actuality, since the image displayed on the display is based upon the three-dimensional information containing noise information generated at the time of CT imaging, it will never be similar to FIG. 1. This point will be described later, so it is explained using FIG. 1 for now.

The three dimensional information shown in FIG. 1 is recognized as the calyx on the display by an operator; however, as described above, information is corresponded per voxel, which is a unit of image display, and is merely displayed on the world area, and as information composition, this is not discriminated from information between the occlusal plane and other calyx at all, and the positional information of the occlusal plane is not even formed. Therefore, the present embodiment adopts a method for detecting an occlusal plane from non-discriminated three-dimensional information by designating an image section, which can be visually confirmed as the positioning member 10 from the displayed image.

First, as it is obvious from FIG. 1 and the description, in the positioning member 10, because its plate member 10a is arranged along the occlusal plane and the globular bodies 10b are arranged in a protrusion manner, it is the same that the globular bodies 10b are arranged on the occlusal plane. Further, the number of the arranged globular bodies 10b is three. Therefore, if a position of the central point of each globular body 10b (the world coordinate system) can be detected, three points on the occlusal plane can also be detected, and then, this results in the detection of the position of the occlusal plane. Herein, detection of a position of the plate member 10 itself can be considered for detecting the occlusal plane. However, the closer [the distance to] the human body becomes, the more the noise (described below) is contained in the actual three-dimensional information obtained by CT imaging, and it can be overlapped with human body elements, such as a tooth alignment, so it is difficult to visually determine whether it is an image from the positioning member 10 or an image based upon other information. Therefore, as a preferable composition, the present embodiment adopts a method for using the globular bodies 10b arranged at a position, which is away from the human body element, so it is difficult for information other than the positional member 10 to be mixed (therefore, the globular bodies 10b are protruded from the plate member 10a).

Next, steps of operation by an operator are described. The detection of the center position of the three globular bodies 10b in order to detect an occlusal plane has been described above; however, as an actual step, an operator designates an area to be detected from the image area where the globular bodies 10b are displayed (STEP 10). This is because the globular bodies 10b have a portion(s) that needs to be secured to the plate member 10a, and that will be embedded into the plate member 10a, and not only the positional information of the globular bodies 10b but that of the plate member 10a is also mixed in this portion, so it is preferable to extract only the information of the globular bodies 10b protruding outward from the plate member 10a. Next, the operator designates a voxel in the vicinity of center point of the image visually confirmed as the globular body 10b (STEP 12). The voxel designated herein becomes a center of the detection area. When the center point of detection is designated, its adjacent point is also detected, and points adjacent to the detected adjacent point are further detected sequentially (STEP 14). These adjacent points may be an adjacent voxel or a voxel separated at a predetermined distance. These adjacent points are detected within the detection area, and when the detection reached outside of the detection area, the process moves on to the next step (STEP 16). Next, the points detected within the detection area are set (STEP 18). This results in the setting of multiple points on the surface of the globular body 10b within the detection area. Furthermore, this is not shown in STEP 18, but it is preferable to contain a step for marking (image display) the set points with a predetermined color(s). This is for visually confirming by the operator whether or not the detection area reaches a positional region other than the globular body 10b; i.e., reaches the positional regions of the plate member 10a or the human body element, and this can be a guideline to determine whether the area designation is good or bad in STEP 10.

Further, when the points of detection are set (STEP 18), two normal lines between each point of detection, respectively (STEP 20), and an intersection position of the detected normal lines are further detected. This is a step for detecting a center position of the globular body 10b using the principle where a normal line between two points on the spherical surface passes through the center of the globe. In this meaning, two normal lines to be detected are ideally sufficient; however, in actuality, there are cases that the point of detection is not positioned on the surface of the globular body or an error is generated due to noise, so it is necessary to detect several normal lines and their intersecting points, respectively. Therefore, next, a step for approximating the actual center position of the globular body 10b by averaging the positions of detected multiple intersection points is required (STEP 24). According to the above steps, one center position of one globular body 10b (world coordinate system) is detected.

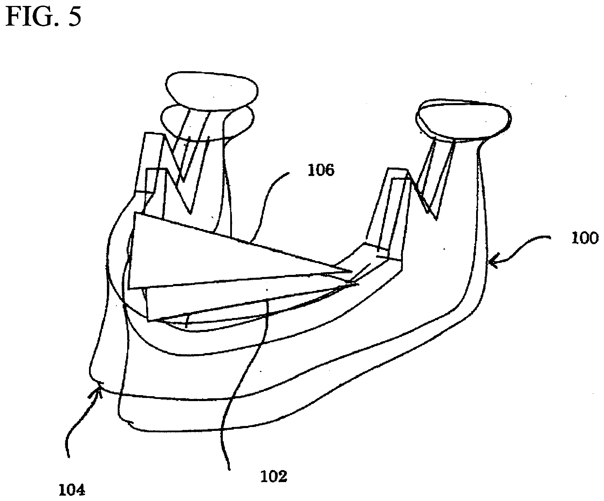

In addition, as described above, the occlusal plane is detected according to at least the center position of the three globular bodies 10b, so after the center positions of the globular bodies 10b are detected, respectively, whether or not the center point of the three globular bodies is detected (STEP 26), and if this is not detected, the steps for detecting other center positions of the globular bodies 10b (STEP 12 to STEP 24) will be repeated. Then, when the center position of the three globular bodies 10b is detected, an occlusal plane is detected (STEP 28). Furthermore, it is not shown, but it is preferable that the detected occlusal plane is displayed with a predetermined color(s) in order for the operator to visually confirm this, and for example, displaying of the occlusal plane using a triangular plane can be considered (refer to Nos. 102 and 106 in FIG. 5 to be used in the explanation in the below-described invention).

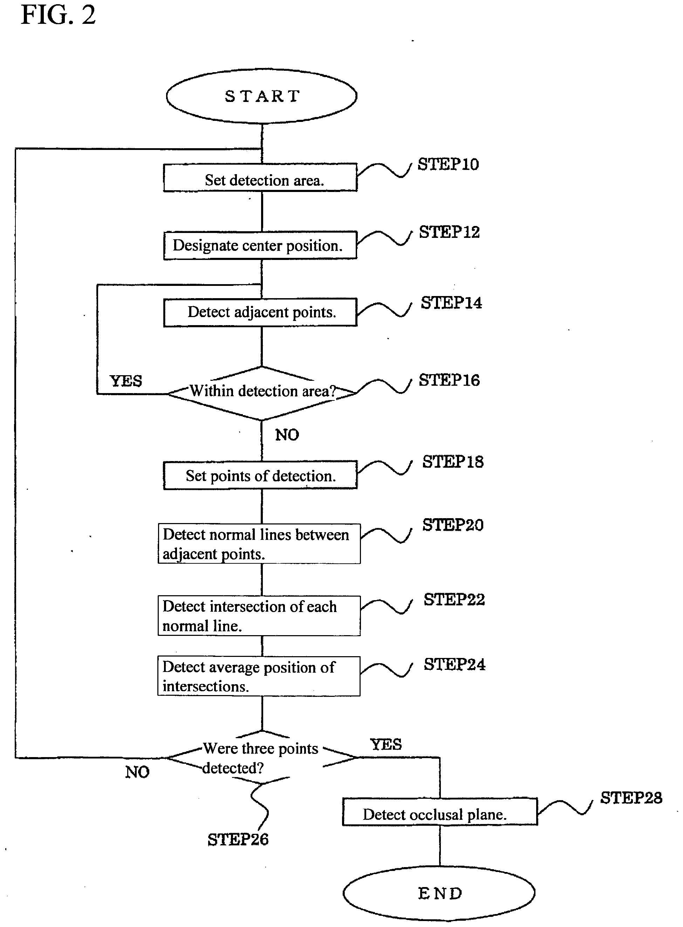

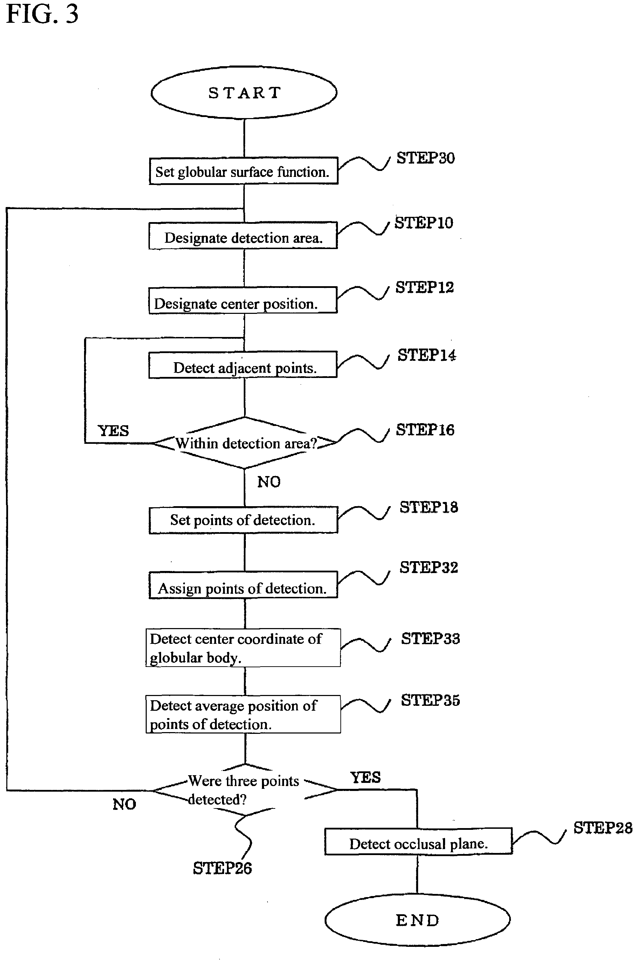

In the detection of the occlusal plane, the method for utilizing the mathematical principle where a normal line between the two points on the spherical surface passes through the center of the globe is disclosed; however, another method for utilizing another mathematical principle can be considered. Specifically, a principle to set the globular surface position with a generalized function (x, y and z coordinates are converted into a parameter using a globular center position and a trigonometrical function), and to detect the globular center coordinates (world coordinate system) by assigning the actual spherical coordinates is utilized. With reference to FIG. 3, the detection steps are shown. In these steps, there are many similar steps as in FIG. 2, so the similar steps are marked with the same reference Nos. as in FIG. 2. Only steps different from the ones in FIG. 2 herein, and first, the generalized function on the spherical surface is set (STEP 30). This step may be comprised as a step between STEP 18 and STEP 32. Further, in STEP 32, the points of detection on the surface of the globular body 10b are assigned to the function set at STEP 30 as a variable, and the center coordinates of the globular bodies 10b are detected by calculating an unknown constant in the function (STEP 33). Then, it is similar to FIG. 2 to resolve an error by averaging the detected center coordinates.

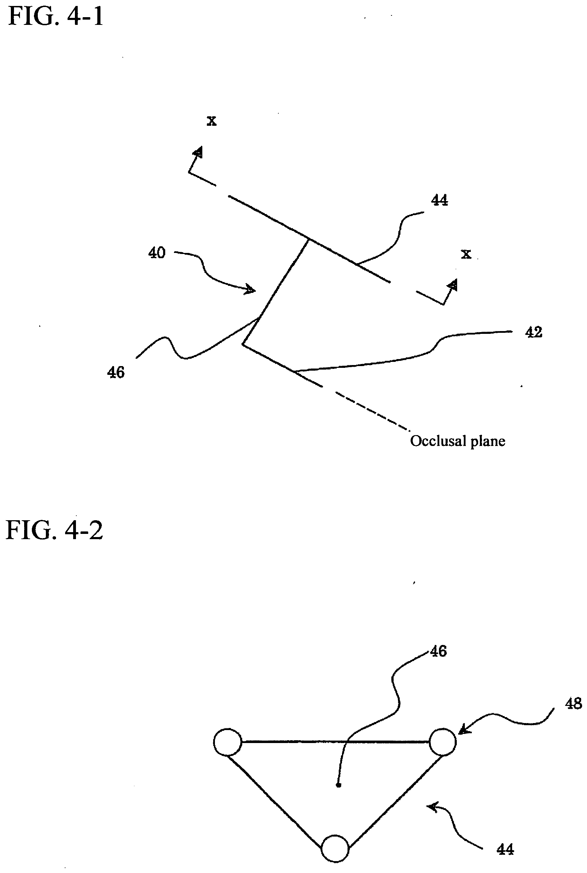

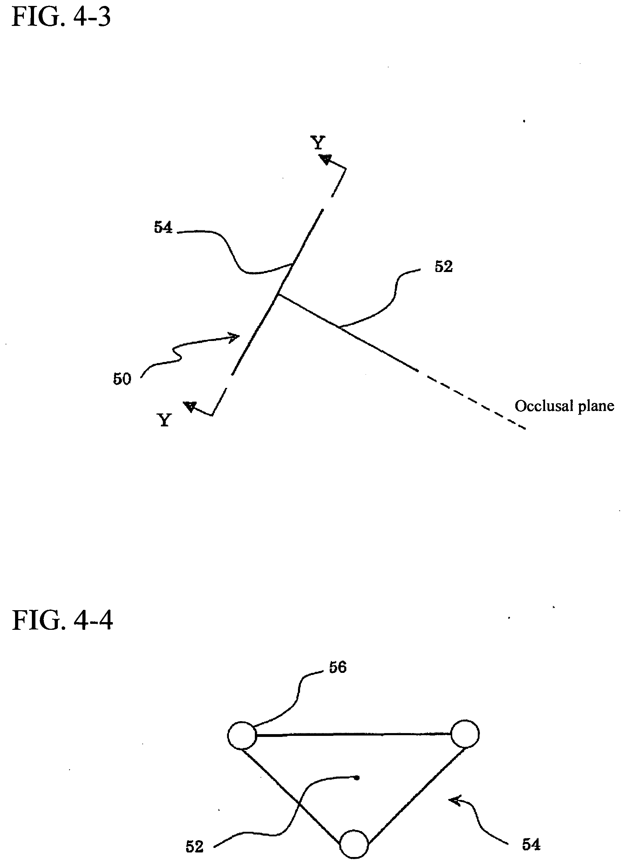

The detection method for an occlusal plane has been described, and for the positioning member used at the time of CT imaging, one other than the one composed of the plate member 10 and the globular bodies 10b (these can be semi-globular bodies as a projection part) are arranged at the sides as shown in FIG. 1 can be considered. This is because anything is acceptable as long as the positioning member can detect a plane relatively secured to an occlusal plane (a reference plane of human body element). The one whose detection plane is situated away from the calyx is rather preferable if considering noise. Specifically schematically viewing, in FIG. 4-1, a second example of positioning member 40 viewing from the side with regard to the occlusal plane, and FIG. 4-2 roughly shows a construction (X-X view) viewing a position detection plane 44 from the upper side. Further, in FIG. 4-3, a third example of the positioning member 50 viewing from the side with respect to the occlusal plane, and in FIG. 4-4, a construction (Y-Y view) viewing a positional detection plane 54 from the upper side. As shown in FIG. 4-1 and FIG. 4-2, the positioning member 40 is composed of an engagement member 42, a detecting member 44 arranged in parallel to the occlusal plane, and a member 46, such as a stick connecting these. With this construction, it is sufficient that the detecting member 44 has a configuration to detect at least a triangular plane, so this can be composed not only with a plate member but with three stick members. Therefore, because globular bodies 48 arranged at the vertices of triangular plane are separated from the calyx and they are less affected by noise, and a portion to be implanted into the detecting member 44 in parallel to the occlusal plane, it becomes possible to more precisely detect an occlusal plane. Further, as the positioning member 50 shown in FIG. 4-3 and FIG. 4-4, an occlusal plane may be detected by detecting a detection member 54, which is perpendicular to the occlusal plane and is fixed to a predetermined positional relationship. Even in this case, as similar to the positioning member in FIG. 4-1 and FIG. 4-2, it becomes possible to detect an occlusal plane with high accuracy while an effect of noise is restrained.

Once the occlusal plane is detected as described above, if the three-dimensional human body information (herein, calyx information) positioned at any position in the world area is set as a reference plane of the positioning of the human body element, it becomes possible to detect a relative position of said human body element. However, herein, another invention (equivalent to the human body information extractor of the present invention) is further provided using the detection method for an occlusal plane as this reference plane (a step by the human information extractor). Hereafter, the specific embodiment is illustrated.

Ideally, if the occlusal plane is detected and the three-dimensional information is converted using this plane as a reference plane, it becomes possible for an operator to designate and to acquire information of a desired site from a three-dimensional image while visually confirming the calyx on the display. However, as described above, it is normal for the three-dimensional information obtained from the CT image to contain noise information, such as artifact, other than the information based upon the human body element, which is a subject to imaging. Therefore, it is unknown whether or not the displayed image has originated from the subject human body element, and in the case of containing a lot of noise information, it is assumed that even an outer image of the human body element cannot be clearly viewed. Therefore, it is required to remove the noise information using some type of method. In the meantime, because tooth alignment models are formed from the same material, even if these are CT-imaged and converted into three-dimensional digital information, noise information, such as artifact, is hardly contained. Noticing this point, a human body information extractor where noise information is removable from human body information using an occlusal plane (reference plane) adopted in the human body information extractor is invented. An embodiment of this device is described as follows:

FIG. 5 shows that two occlusal planes detected from two three-dimensional imaged information is being overlapped are schematically viewed. First, the calyx 100 at the right side represents a three-dimensional image (three-dimensional information) obtained from the CT image of a patient. This is an image arranged within the world area, and a step for detecting an occlusal plane is as described above. In FIG. 5, the detected occlusal plane is represented with a reference No. 102 (because the occlusal plane 106 described below is overlapped [the occlusal plane 102], it is visually confirmed as trapezoid; however, it is actually a triangular plane). Next, the calyx 104 shown at the left side is a three-dimensional image obtained from the CT image information of the tooth alignment model of the patient. This image is arranged as one unit of object within the world area, and the image of the calyx 104 is set so as to be displaced relative to the world area. Therefore, the image of the calyx 104 is displaced (moved or rotated) associated with the displacement of the object within the world area.

The occlusal plane 106 is detected at the step regarding the three-dimensional information from this tooth alignment model, as well. In the human body information extractor, the three-dimensional image (the calyx 100) is displaced within the world area where the three-dimensional image (the calyx 104) is obtained from the patient information, and it is apparently positioned at a position where both the calyx planes 102 and 106 are matched with each other, respectively. At this time, in the case that the sizes of the triangular planes 102 and 106 indicating an occlusal plane are different, the calyx 100 or the calyx 104 is expanded or reduced so as to have the same area size, and the triangular planes 102 and 106 are attempted to be totally matched. Next, once the occlusal planes 102 and 106 are matched, the three-dimensional human body information at the position corresponding to the three-dimensional image of the tooth alignment mold is detected. Then, the detected human body information (image information and other medical information) is extracted, and it is set as human body information to be used for processing thereafter.

As described herein, if taking the three-dimensional information obtained from the tooth alignment model, which is formed in a solid-core state with the same material, into consideration, the image becomes an outline display of the human body element without any noise information (or with less noise information). Therefore, if the occlusal planes 102 and 106 are matched, the images of the calyxes 100 and 104 should be matched on the outline display. However, in actuality, a portion(s) that does not mach exists, and this is a displayed image caused by the noise information generated at the time of CT imaging. Therefore, in the present embodiment, the detection and setting of only the human body information corresponding to the tooth alignment model among the three-dimensional information obtained from the CT image information enable the extraction of the three-dimensional human body information without any noise information.

Next, the calibration of CT values in the CT image information of a patient is described. The CT image information is known that the information of a site in the patient (human body element, meaning the maxilla and the mandible especially in the present embodiment) on the occasion of displaying with gray-scale. This gray-scale is an image display based upon numerical numbers according to dimensionless X-ray absorption of the site in a patient (in detail, substance ingredients contained in said site), and it is referred to as a CT value, respectively. This CT value varies per CT imager and per patient, and it is merely a relative value of the X-ray absorption within the same patient imaged by the same CT imager, but it is not an absolute value. Therefore, a CT value at the same site in other CT image information is not always the same numerical value, and there is a difference in the CT values even in the site of the same section depending upon the CT imager or patient (especially volume difference), so it has a problem where a plurality of CT image information cannot be absolutely compared by using CT values. Consequently, in the medical field, in order to make CT values absolute, an attempt is made to calibrate a CT value of an actually-used CT imager to a specified value (Hounsfield value) that is standardized corresponding to the substance ingredient. This is normally referred to as calibration of CT value in the medical field.

Specifically explaining the conventional calibration, a transparent body where water is poured into the inside is imaged as adjustment before the CT imaging of a patient, and CT values of the water portion and the void portion are detected from the imaged information. Then, these CT values are compared to the Hounsfield value (specifically, - 1024) of void portion and the Hounsfield value (specifically, 0) of water, respectively. Because a difference of the actually measured CT values can be determined from two points according to this comparison, a difference of the entire CT values (a difference from the Hounsfield values) to be generated per CT imager can be detected with a gradient. Therefore, in the medical field, if the CT values of the water portion and the void portion are calibrated to the Hounsfield values, respectively, the calibration of CT values can be accomplished. However, in the medical field, due to complication of the procedures, it is difficult to have CT imaging after the calibration per patient, and in most of cases, the calibration is periodic or none at all. Further, in the conventional calibration, even though it is possible to calibrate differences of CT values to be generated per CT imager, differences of CT values caused by a volume difference per patient cannot be calibrated.

In the meantime, in the present embodiment, even though the calibration is not executed before CT imaging as in the conventional method, it is still possible to execute the calibration from the imaged information (three-dimensional human body information) after the CT imaging. Specifically, a positioning member used in the present embodiment is utilized. For example, a case of using the positioning member 10 is described. As described above, the positioning member 10 is equipped with the globular bodies 10b in order to detect an occlusal plane, and herein, the globular bodies are formed from hollow transparent ceramics having X-ray transmissivity, and water is poured inside. The positioning member 10 having the globular bodies formed as described above is occluded to a patient, and the CT is imaged. Then, a CT value of the globular body 10b in the CT image information and a CT value of a void portion not in the patient (a portion other than the human body element and it corresponds to the void portion around the periphery in FIG. 1 are detected. At this time, as described above, the Hounsfield values of the water component and the void component are already known (0 and -1024, respectively), and these are fixed values regardless of patients and CT imagers. Therefore, a function of the actually measured CT values with regard to the Hounsfield values can be detected from the Hounsfield values corresponding to the CT values of actually measured two points (globular body 10b and void portion) and their corresponding Hounsfield values. When the actually measured CT values at each position of the maxilla and the mandible as the human body information arranged within the world area are sequentially applied to this function, the three-dimensional image information totally converted into the Hounsfield values can be obtained. In other words, in the present embodiment, utilizing the specified positioning member 10b used for the detection of a reference plane (for example, an occlusal plane) of a human body element enables the ex-post calibration from the CT image information regardless of an adjustment difference per CT imager and a difference per patient. Furthermore, in the aforementioned description, the configuration where the material of the globular body is ceramics and water is poured inside is illustrated. However, [the present invention] is not limited to this, and as long as a stable CT value can be detected, other materials are acceptable.

Further, the positioning member 10b is also usable for calibration of distortion at the time of CT imaging. With a CT imager, normally, a patient is moved toward a central axis direction (referred to as Z-axis direction) within the imager on a hollow dome and the patient is imaged. At this time, a method where while an actual CT imager rotates around the patient (the Z axis-wise) at a predetermined pitch, the imager slightly moves relatively toward the Z direction and [the images] are overlapped is adopted (generally, it is referred to as helical scanning, and each pitch is referred to as a helical pitch). When helical scanning is conducted as described above, there is a problem that the image inevitably shifts toward the Z axis direction. For example, the CT image of globular body becomes a slightly oval sphere with the Z axis direction as a major axis. In other words, in the CT image information, the positional information in the Z axis direction will not be precise. In the present embodiment, in order to resolve this problem, the problem in the helical scanning is avoided by calibrating a detected distance between specific portions of the positional member 10, in this case, between the globular bodies 10b to an actual distance. Specifically, from the CT image information, as similar to the execution for the reference plane, center positions of the two globular bodies 10b are detected. According to this detection result, a distance between the globular bodies 10b, especially the distance in the Z axis in the imaged information is detected. Further, an actual distance of the center positions between the globular bodies 10b of the positioning member 10b is also measured, separately. Then, the entire positional information in the CT image information is calibrated so as to match the detected distance in the CT image information with the actual distance. This calibration enables the calibration of any influence by the helical scanning to be generated at the time of CT imaging, by using the positioning member 10 having a known distance.

Further, the positioning member 10 is also usable as a tool for extracting only information at a necessary site from CT image information. For example, on the occasion of detecting an occlusal plane as a reference plane using the positioning member 10 from the CT image information as described above, a predetermined area in the upper side is set based upon the occlusal plane, and only said setting area, for example, only the maxilla can be pre-extracted, or the maxilla and the mandible can be separately extracted based upon the occlusal plane. Therefore, it is also possible to miniaturize or standardize the data size of the CT image information, and the processing thereafter can be simplified.

Next, the fixture at the time of CT imaging of a human body model is illustrated. In the present invention as described above with reference to FIG. 5 and FIG. 1, in order to detect the imaged information of upper and lower tooth alignments 12 and 14 of the calyx 16 from the CT image information (three-dimensional imaging information of human body element), the redundant human body image information is extracted by overlapping the CT image 100 of the calyx 16 onto the CT image 104 of the human body model based upon the occlusal planes 102 and 104 , respectively. However, in actuality, in the case of this processing, the image of the positioning member 10, which has been used for detecting the occlusal planes, also remains. Consequently, after that, in the case of processing, such as implant simulation of an artificial dental root via images, the presence of this positioning member becomes an obstacle to the operator, and it will be an adverse effect on the occasion of attempting highly precise processing by the operator. Consequently, it becomes necessary to efficiently eliminate the image information of the positioning member later.

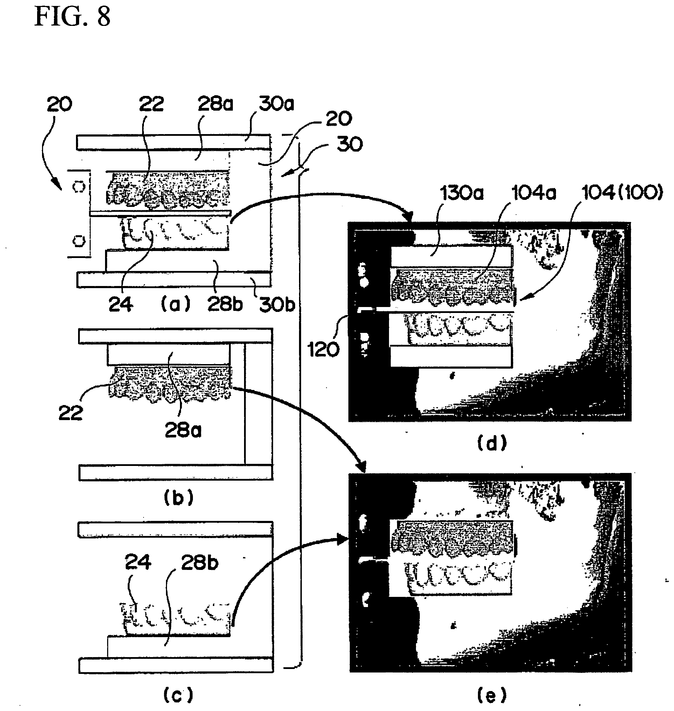

In the present embodiment, the human body information extractor with excellent usability is provided by taking this point into consideration, as well. With reference to FIG. 8, an imaging method for models of upper and the lower tooth alignments and the positioning member, and the fixture at the time of imaging are shown in (a) to (c), and a method for extracting human body information and a method for eliminating the positioning member on the image are shown in (d) to (e). FIG. 8(a) shows a situation where upper and lower tooth alignment models 22 and 24 are fixed to a fixture 30 for model imaging in the state of fitting a model 20 of positioning member. The fixture 30 for model imaging is composed of an upper contact member 30a, a lower contact member 30b and a connector 30c connecting these members. A distance (positional relationship) between the upper and lower contact member 30a and 30b is always constant when the upper and lower tooth alignment models 22 and 24 are fixed, and it is also possible to separate the upper and lower tooth alignment models 22 and 24 as a matter of convenience of work efficiency in a stage for fixing the upper and lower tooth alignment models 22 and 24.

First, as shown in FIG. 8(a), the upper and lower tooth alignment models 22 and 24 are fixed to the fixture 30 for imaging in the condition of being fitting to the positioning member model 20, in other words, in a similar model condition to the one where a patient is CT-imaged. At this time, it is possible to maintain the state where the upper and lower tooth alignment models 22 and 24 come into contact with the upper and lower contact members 30a and 30b in gaps between the upper and lower contact members 30a and 30b of the fixture 30 for imaging and the upper and lower tooth alignment models 22 and 24, respectively; concurrently, flexible materials 28a and 28b are intervened so as to easily enable separating the both members. The CT is imaged in the state of FIG. 8(a). Then, the model image and the patient image are fitted based upon the occlusal planes with the technique showing in FIG. 5 and in the descriptions for this, and only desired patient information is extracted. The fitted image is shown in FIG. 8(d).

However, as shown in FIG. 8(d), an image 120 of the positioning member 10 and images 130a and 130b of the flexible materials 28a and 28b still remain. Therefore, after that, a user who executes various processes with visual confirmation of the images will desire to mark only the upper and lower tooth alignments and to delete the images of the positioning member in order to improve the workability. In order to satisfy this user request, in the present embodiment, after the desired human body information is extracted by fitting shown in FIG. 8(d), the model information is temporarily deleted from the image display.

Next, as shown in FIG. 8(b) and (c), only the upper and lower tooth alignment models 22 and 24 are CT-imaged, respectively. With this process, single model images of the upper and lower tooth alignment models 22 and 24 having the same positional relationship as that in FIG. 8(a) can be acquired even though the positioning member model 20 does not exist. As shown in FIG. 8(e), these model images are overlapped onto the image where model images have been eliminated after fitting shown in FIG. 8 (d). On the occasion of this overlapping, since the images in FIG. 8(b) and (c) have the same positional relationship as that in FIG. 8(a), both images will be perfectly matched even on the display, by merely overlapping the images in the same positional relationship as that in FIG. 8(d). Therefore, if this overlapping processing is executed, it becomes possible to mark only a site desired by a user, such as the upper and lower tooth alignments (maxillary and mandibular tooth alignments), and to eliminate any obstacle image, such as the positioning member 120.

In the aforementioned embodiment, the method for acquiring the three-dimensional image information based upon a reference plane from the CT image, which is imaged using the positioning member [model] 20, that matches the human body reference plane (such as an occlusal plane) having the positional relationship relative to the maxilla and mandible has been explained. However, it is also possible to use the fixture 30 as an alternative of the positioning member [model] 20 without using the positioning member [model] 20. Hereafter, the embodiment is described. Herein, similarly to the description of the aforementioned embodiment, an example of CT image acquisition based upon an occlusal plane, which is one of the reference planes in the dental treatment, is described.

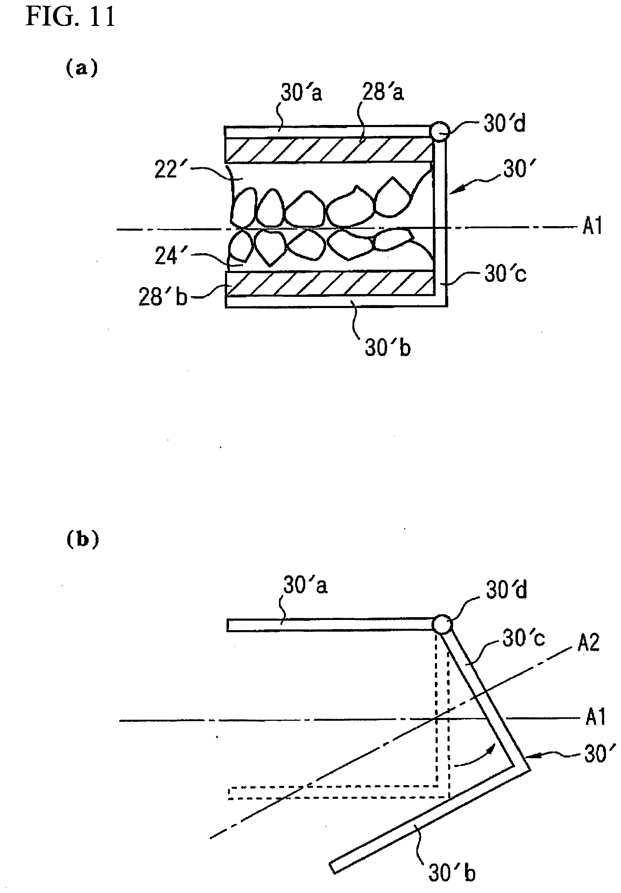

FIG. 11 shows an imaging method for models of upper and lower tooth alignments and a fixture for fixing the upper and lower tooth alignment models and acquisition of an occlusal plane. Among the reference Nos. in this view, the same reference Nos. shown in the aforementioned descriptions and FIG. 8 mean the same types, and for example, a fixture 30' is a modification example of the fixture 30 for model preparation. FIG. 11 (a) shows a situation where upper and lower tooth alignment models 22' and 24' are occluded and fixed to the fixture 30'. Further, FIG. 11 (b) shows a situation where a junction member 30'c pivots and the fixture 30' is opened (as described below, both the upper and lower tooth alignment models 22' and 24' are also fixed to the upper and lower contact members 30'a and 30'b, respectively; however, herein, taking the easiness of visual confirmation into consideration, illustration is omitted). First, the fixture 30' is composed of the upper contract member 30'a & the lower contact member 30'b, the connecting member 30'c for connecting these members, and a hinge 30'd for connecting the upper contact member 30'a and the connecting member 30'c to be pivotable. Therefore, because the length of the connecting member 30'c is fixed, in the state where the upper and lower tooth alignment models 22' and 24' are occluded as shown in FIG. 11 (a), a distance (positional relationship) between the upper and lower contact members 30'a and 30'b is constant; however, as shown with the solid line in FIG. 11 (b), in the case of pivoting the lower contact member 30'b downward centering on the hinge 30'd, the upper and lower contact members 30'a and 30'b are released (shown), and the upper and lower tooth alignment models 22' and 24' to be fixed to these members are also released from each other (not shown). At this time, since the connecting member 30'c and the lower contact member 30'b are fixed with each other, the connecting member 30'c and the lower contact member 30'b pivot in the same positional relationship.

Further, FIG. 11 (a) shows a situation where the tooth alignment models 22' and 24' are fixed in the case of a patient having tooth alignments with normal occlusion, and the fixation of the upper and lower tooth alignment models 22' and 24' to the fixture 30' is positioned so as to have the upper and lower tooth alignments occluded. As similar to the case in FIG. 8, this positioning is conducted by inserting flexible materials 28'a and 28'b so as to enable maintaining the state where the upper and lower tooth alignment models 22' and 24' come into contact with the upper and lower contact member 30'a and 30'b within gaps of the upper and lower contact members 30'a and 30'b of the fixture 30' and the upper and lower tooth alignment models 22' and 24', respectively; concurrently, to be easily separable. With this positioning as described above, in the case of FIG. 11 (a), as shown in dashed-dotted lines A1 and A2 , the occlusal planes on the model can be detected at a positions relatively fixed to the fixture 30'. These occlusal planes A1 and A2 mean occlusal planes of the maxilla and mandible, respectively, and in the state showing in FIG. 11 (a), because the upper and lower tooth alignments are occluded, each occlusal plane (the maxillary occlusal plane A1 and mandiblular occlusal plane A2) is also overlapped. Further, in this fixture 30', since the occlusal plane is detected not by occluding the positioning member [model] 20 with the tooth alignment models as shown in FIG. 8, even in the case that occlusion cannot be normally conducted, such as when many portions are missing from tooth alignments, it is possible to detect the occlusal planes of the maxilla and the mandible. Specifically, as shown in FIG. 11 (b), while the upper and lower contact member 30a' and 30b' pivot centering on the hinge 30d' by an operator, such as a dentist, in a state where the tooth alignment models 22' and 24' are fixed, he/she visually confirms the upper and lower tooth alignment models 22' and 24', and authorizes (assumes) the occlusal position. Then, when the occlusal position is authorized, the hinge 30'd is fixed. At this time, the maxillary occlusal plane A1 and the mandibular occlusal plane A2 are planes in parallel to the upper contact member 30'a and the lower contact member 30'b, respectively, and the relative position between the upper and lower contact members 30a' and 30b' is fixed except for a pivot direction centering on the hinge 30'd, the mandibular occlusal plane A2 is also positioned relative to the maxillary occlusal plane A1 except for the pivot direction. In other words, if this fixture 30' is used, regardless of the tooth alignment condition in a patient, the maxillary occlusal plane A1 and the mandibular occlusal plane A2 can be detected based upon the upper contact member 30'a, respectively.

Next, in the present embodiment, CT of the fixture 30', where the tooth alignment models are fixed in the state of FIG. 11 (a), is imaged. Then, an image of the fixture 30' including a model image (model image of a patient) and the patient image are fitted by a technique similar to that described in Fig. 5 and Figs. 8(d) to (e) using the positioning member [model] 20. In the method shown in Fig. 8(a), as described above, the CT images of the upper and lower tooth alignment models 22 and 24 are overlapped onto the corresponding portions of the CT image of the patient, the redundant image information is extracted as the positional information relative to the positioning member [model] 20; however, in the present embodiment, the image of the upper contract member 30'a of the fixture 30' is overlapped onto a camper plane within the image of the patient. Herein, the camper plane is normally used as a reference plane of the human body by dentists, and it is a plane connecting predetermined positions of the sub-nasal and the otic within a patient. This camper plane is in parallel to the maxillary occlusal plane.

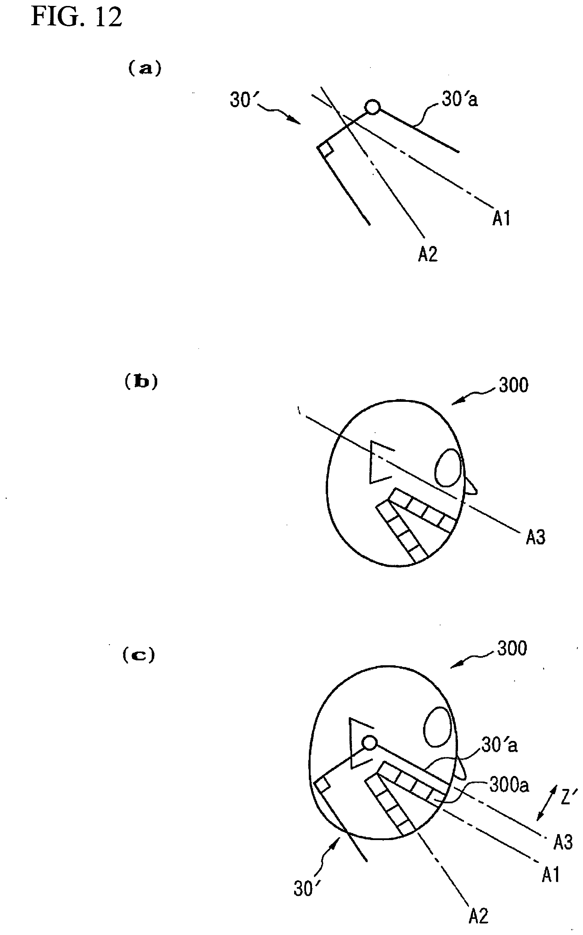

Herein, with reference to Fig. 12, each view is a schematic view showing a situation where the CT image 300 of a patient is fitted to the CT image of the fixture 30'. FIG. 12 (a) shows the CT image of the fixture 30' (taking the easiness of visual confirmation into consideration, the CT images of the upper and lower tooth alignment models 22' and 24' fixed to the fixture 30' are omitted); Fig. 12 (b) shows the CT image 300 of the patient; and Fig. 12(c) shows that the CT image of the fixture 30' shown in Fig. 12 (a) is overlapped onto the CT image 300 of a patient shown in Fig. 12 (b). In these diagrams, the dash-dotted lines A1, A2 and A3 represent a maxillary occlusal plane, a mandibular occlusal plane and a camper plane, respectively. As described above, since the maxillary occlusal plane A1 is positioned in parallel to the upper contact member 30'a at a predetermined distance, if the upper contact member 30' on the CT image of the fixture 30' is matched with the camper plane A3 on the CT image 300 of the patient, the maxillary occlusal plane A1 in parallel to the camper plane can be detected. At this time, a distance between the upper contact member 30'a on the CT image of the fixture 30' and the maxillary occlusal plane A1 may shift from a distance between the camper plane A3 and the maxillary occlusal plane A1 on the actual CT image 300 of the patient according to a change of thickness of the flexible member 28'a of the fixture 30', and this will be corrected by vertically (Z' direction in Fig. 12(c)) moving the image information of the upper contact member 30'a toward the maxillary occlusal plane A1. At this time, fitting of the tooth alignment model 22' (not shown in Fig. 12) to the tooth alignment 300a on the CT image 300 of the patient enables the correct determination of the travel distance along the Z' direction.

Further, since the mandibular occlusal plane A2 is also positioned in the predetermined positional relationship with the upper contact member 30'a (described above), if the CT image of the upper contact member 30'a is overlapped onto the camper plane A3 of the CT image of the patient, the maxillary occlusal plane A1 and the mandibular occlusal plane A2 in the CT image of the patient can be detected.

Therefore, the CT image 300 of the patient can be regarded as a CT image based upon the maxillary and mandibular occlusal planes A1 and A2, respectively. Consequently, even if a patient is CT-imaged in any posture, imaged information using an occlusal plane that is easily understandable to a dentist as a reference can be acquired. Further, even in a patient where because portions missing from a tooth alignment(s) are great, he/she cannot have normal occlusion, the CT image information using the occlusal plane as a reference can be easily acquired. In addition, since the fixture 30' in the embodiments shown in Fig. 11 and Fig. 12 has a function similar to a known occludator (occlusal plane confirmation device) referred to as an average value occludator, it is easy for a dentist who normally uses this to use this occludator.

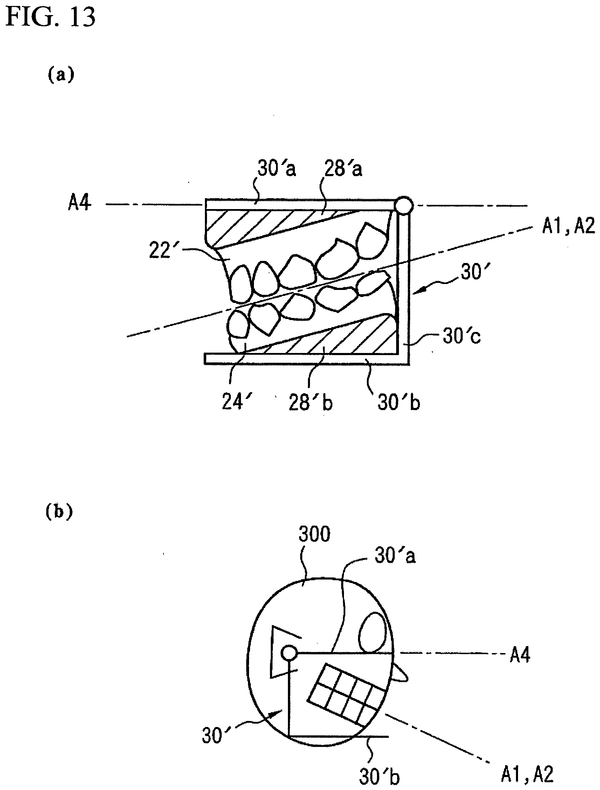

Further, in the embodiments described with reference to Fig. 11 and Fig. 12, it is designed to detect an occlusal plane based upon the camper plane of the upper contact member 30'a. In the meantime, an eye-ear plane (Frankfort horizontal plane) is also available as a reference plane of the human body to be used other than a camper plane by a dentist, and the eye-ear plane is a plane connecting portions under the eye and the ear, and an occlusal plane is detected based upon said plane with a known occludator referred to as an adjustable occludator. In the present embodiment, an occlusal plane can also be detected using this eye-ear plane as a reference. With reference to Fig. 13 (a), the tooth alignment models 22' and 24' are positioned at the fixture 30' similar to Fig. 11 (a), and the elements marked with the identical reference Nos. to those in Fig. 11 and Fig. 12 represent the same, respectively. At this time, in the case of Fig. 11 (a), the tooth alignment models 22' and 24' are fixed via the flexible materials 28'a and 28'b so as to position the maxillary and mandibular occlusal planes A1 and A2 in parallel to the upper and lower contact members 30'a and 30'b, respectively; however, in the embodiment shown in Fig. 13 (a), the tooth alignment models 22' and 24' are fixed by the flexible materials 28'a and 28'b so as to position the maxillary and mandibular occlusal planes A1 and A2 by matching the eye-ear plane A4 with the upper contact member 30'a. Furthermore, herein, [the embodiment] is described on the assumption that the maxillary and mandibular occlusal planes A1 and A2 are matched; however, if the both planes are not matched, they should be positioned by pivoting the fixture 30', so the description of the embodiments, which have already been shown in Figs. 11 and 12, should be referred.

The fixture 30'a shown in Fig. 13 (a) configured as described above is CT-imaged, and the image is overlapped onto the CT image 300 of a patient as shown in Fig. 13 (b) (refer to as Figs. 12 (a) to (c), as well). At this time, the image of the upper contact member 30'a among the CT image of the fixture 30' is matched with the eye-ear plane A4 within the CT image 300 of the patient. At this time, [planes] are detected as an upper and lower occlusal planes on the CT image 300 of the patient to match the maxillary and mandibular occlusal planes A1 and A2 positioned relative to the fixture 30'. Therefore, it becomes possible to acquire the CT image of a patient based upon the occlusal planes even using the eye-ear plane A4 as reference, and since the fixture 30' of the embodiment shown in Fig. 13 has a function similar to the known occludator (occlusal plane confirmation device) referred to as an adjustable occludator, this is very useful for a dentist who normally uses this occludator.

In addition, in the description, regarding the fixture 30' shown in FIGS. 11 to 13, it is preferable that the upper and lower contact members 30'a and 30'b, the connecting member 30'c and the hinge 30'd, which are its components, are formed from a material with high X-ray transmissivity, such as acryl. The CT image 300 of a patient contains a lot of noise information (images) referred to as artifact, and this will be an obstacle on the occasion of directly detecting an occlusal plane from the CT image, so [the elimination of this information] will be a necessity for fitting. In particular, a material with low X-ray transmissivity, such as metal materials, will be greatly affected by the artifact. In the meantime, this fixture 30' has an advantage that it is easy to be fitted onto the CT image of a patient because the influence by the artifact is less and the tooth alignment models 22' and 24' are clearly displayed.

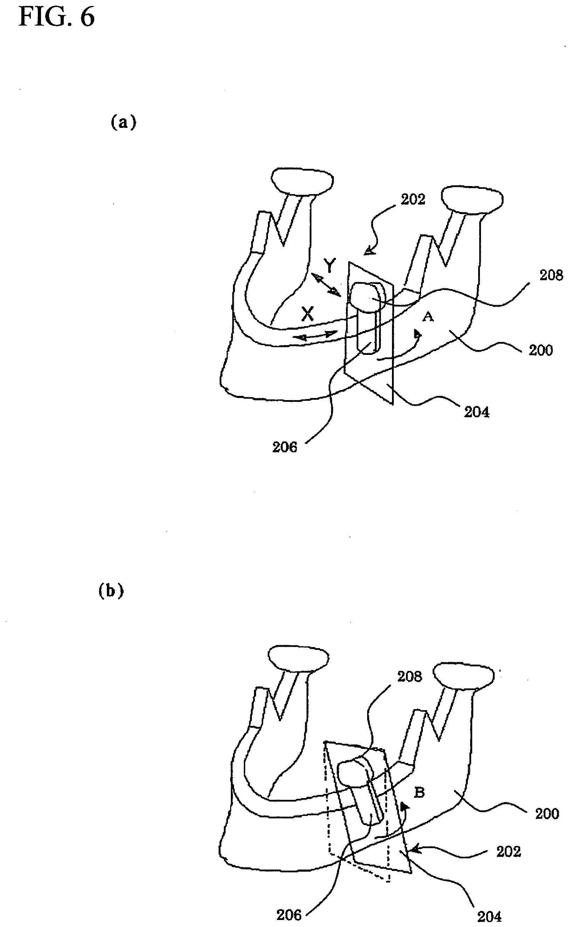

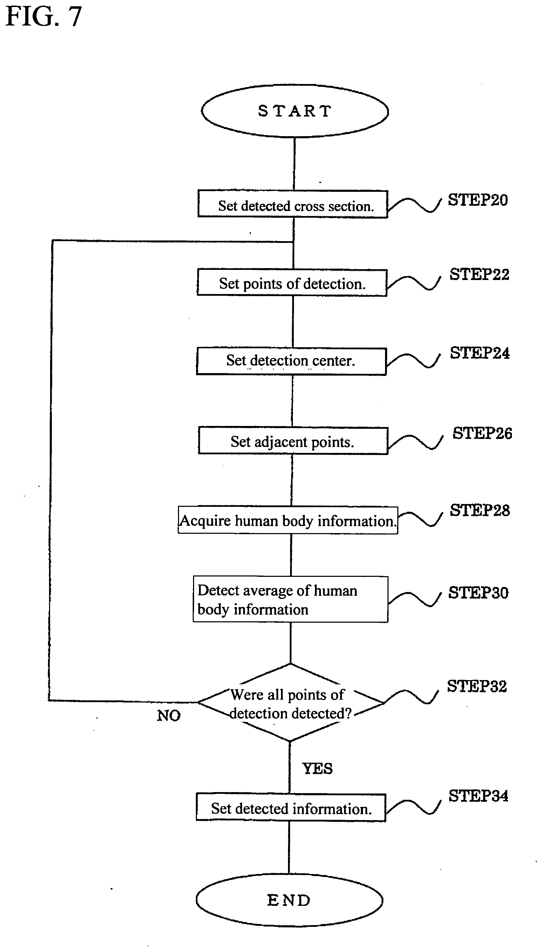

Next, a device for detecting cross-section information of three-dimensional image based upon the human body information extracted from the human body information extractor is described. Fig. 6 shows steps processed by this device with images on the display, and Fig. 7 shows a flowchart showing these steps. First, describing the images on the display, as shown in Fig. 6 (a), the calyx 200 is displayed on the display based upon the three-dimensional information obtained from the CT imaging. This displayed image is arranged on the world area. An operator recognizes a portion (not shown) missing from a tooth alignment by visually confirming this image. Once the missing portion is recognized, first, the operator attempts to fit a dental crown image 208 into the missing portion.

Herein, fitting of the dental crown image 208 is described. For the dental crown image 208, CT of a formed dental crown model is pre-imaged, and [the dental crown image 208] is arranged at any position on the world area as one object based upon the imaged information. Normally, this image (object) is moved on the world area by the operator, and the image is arranged at a position of the missing portion determined as appropriate for overlapping. However, there is another case that a patient has many portions missing from a tooth alignment(s), and if many dental crown images 208 are arranged at any positions separated from the missing portions before the objects are moved, it becomes difficult for the operator to determine which one is for each missing tooth alignment, and it is possible to lower the processing efficiency. Consequently, in the present embodiment, it is designed such that the dental crown images are automatically arranged in the vicinity of the defect tooth alignments, respectively, so it makes clear which dental crown images should fit into the missing tooth alignments. Specifically, a reference plane (occlusal plane) detected from the positioning member 10 is utilized. In detail, first, the tooth alignments are pre-set on the detected occlusal plane; and dental crown image information is arranged on said tooth alignments, respectively, but each dental crown image is not displayed on the display. Then, in the stage for overlapping the dental crown images onto the missing portions by the operator, the correspondent dental crown images 208 on the occlusal plane are displayed onto the appropriate missing portions. With this processing, even in the initial stage before the dental crown images are moved, the dental crown images desired by the operator are automatically displayed at the positions close to the missing portions to some extent, and in the meantime, it can be designed that not-desired dental crown images will not be displayed.

Next, processing after visually confirming that the dental crown image 208 is fitted into the defect portions is described. An implant 206 is displayed along its reference axis at the lower end of this dental crown image 208, and in the reference axis 206, a predetermined plane area 204 including said axis is attached to the reference axis 206.

Therefore, when the operator moves and positions the dental crown image 208 on the world area so as to fit into a portion missing from the tooth alignment, the implant and the reference axis 206 are also positioned according to said positioning. In addition, when the reference axis 206 is positioned, the plane area 204 is also positioned. This plane area 204 is a plane for desired cross-section information. Herein, mentioning the position of the plane area 204, if a local area where an image 202 for detecting a cross section image is arranged is set within the world area, a dental crown image position, an angle of inclination of the reference axis 206 and an angle of rotation of the reference area 204 can be set. Fig. 6 (a) roughly shows a state where rotation of the reference axis 206 within the world area using the reference axis 206 as the Z axis results in the rotation of the reference axis along the arrow A direction, and associated with this rotation, the implant (accurately, major axis of implant) 206 and the plane area 204 are rotated. On this occasion, if the local coordinate system where the reference axis 206 and the plane area 204 are regarded as the Z axis and the XY plane, respectively, is set, three-dimensional imaging information in the world coordinate system corresponding to said [local] coordinate system will be desired cross-section information on the plane area 204. Further, Fig. 6 (b) schematically shows a state where inclination of the local coordinate system based upon the reference axis 206 with regard to the world coordinate system results in inclination of the reference axis in the direction of arrow B, and associated with this inclination, the implant 206 and the plane area 204 are rotated. If human body information on the world area corresponding to the human body information of the plane area in the local coordinate system positioned as described above is acquired, the human body information on said plane area can be acquired. Therefore, with the present device, while the positions of the dental crown and the implant to be implanted are confirmed, it becomes possible to acquire the cross-section information.

The aforementioned is a description of the cross-section information extractor of the present invention, and one example of the steps specifically processed by this device is also described. As shown in Fig. 7, rotation and inclination of the reference axis by an operator as described above results in the setting of the plane area 204, i.e., desired detected cross section (STEP 20). Next, points of detection within a detection cross section are set. Specifically, a voxel situated at a pre-set position is detected from many voxels arranged on the detection cross section, respectively, and this is set as a point of detection (STEP 22). Next, one point of detection is set from multiple set points of detection in the plane area as a center of detection (STEP 24). In addition, an adjacent point positioned in the vicinity of the center of detection is detected and set (STEP 26). The adjacent point to be set in this step is executed by detecting a point (voxel) situated within the pre-set area (voxel group area) from the center of detection. Then, human body information on the world area corresponding to the set center of detection and adjacent point is acquired (STEP 28), and a plurality of the acquired human body information is averaged (STEP 30). In addition, whether or not the human body information corresponding to the center of detection is detected (acquired) throughout the entire plane area is determined, and STEP 22 to 30 will be repeated until it is detected (STEP 32). Then, when the human body information is detected throughout the entire plane area, setting of this information on a separately set screen as two-dimensional information (STEP 34) enables the provision of cross-section information around the reference axis, such as an implant, to the display. Furthermore, in Fig. 7, even though steps for acquiring the human body information per detected area and for averaging [the information] are shown, a step for averaging the human body information in the detected areas having each center of detection and adjacent point after acquiring the human body information at each point of detection throughout the entire plane area is also acceptable. Specifically, it is considered inserting STEP 28 and STEP 32 between STEPS 22 and 25.

Furthermore, it is also possible to measure a distance between any two points on the plane area 204 or to measure an angle among any three points. Specifically, coordinates of desired two or more points of position detection (voxels) on the world area are specified, and when the number of the specified points of position detection is two, a distance between the detection points is measured using the points of detection and their coordinates. Further, when the number of the specified points of position detection is three or more, line segments formed with any two points among the points of detection, respectively, are measured from the coordinates, and an angle of said line segments and/or a distance between the line segments are measured using the coordinates of the points of detection.

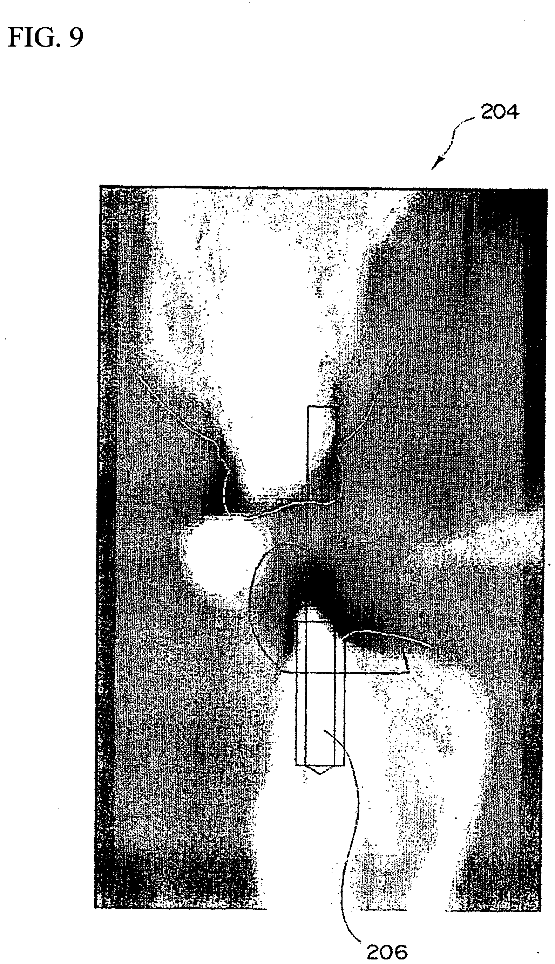

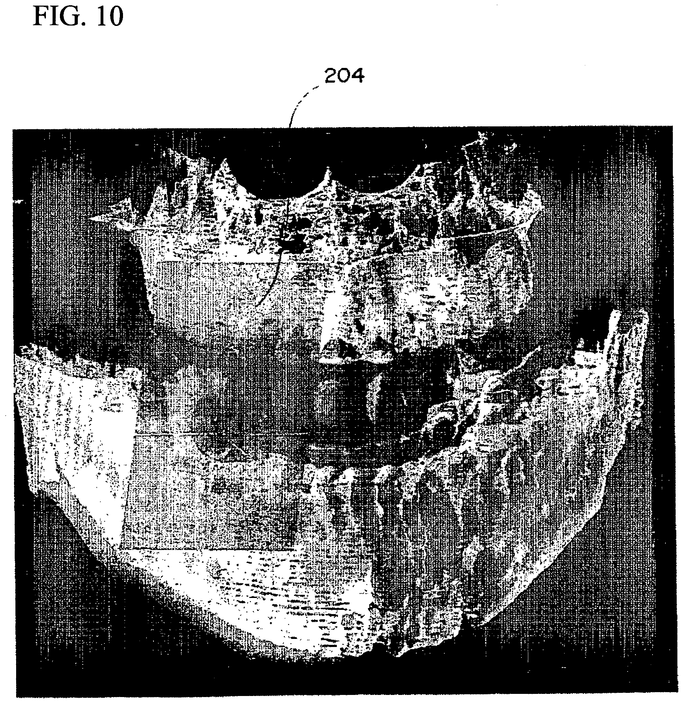

In addition, the cross-section information detector of the present invention also provides another embodiment. In this cross-section information detector, the reference axis 206 can be inclined in a predetermined direction based upon a human body element, and the plane area 204 can be inclined along with the reference axis 206. Herein, the inclination in the predetermined direction of the human body element, for example, means that an inclination in a tooth alignment direction shown as the X direction in FIG. 6 (also referred to as mesiodistal inclination) or an inclination in a buccolingual direction shown as the Y direction. The former is obtained by mesiodistally inclining the vicinity of the upper end of the reference axis 206 (a cervical part in an artificial dental root image) as a center, and the latter is obtained by buccoligually inclining the vicinity of the upper end of the reference axis 206 as a center. The concept of coordinates in this inclination is the one, which is different from the normal coordinates in the world area, but is assumed by a dentist at the time of surgical operation. Herein, the concept of coordinates assumed by a dentist at the time of surgical operation is further mentioned. What is positional reference on the occasion of the surgical operation by a dentist is normally an occlusal plane, and he/she determines the appropriateness of surgical operation using the coordinates of the viewpoint by viewing the condition of the occlusal plane from the front of a patient. Therefore, for the dentist, even if the cross-section information is detected on the coordinate plane of the world area, if he/she attempts to reflect the information to the surgical operation, a process for substantially converting and recalling [the cross-section information] with the coordinates of the viewpoint from the front of the occlusal plane is required. In the present embodiment, focusing upon this point, the conventional process for substantially converting the coordinate system in the mind of the dentist is eliminated, and the process is designed to detect the cross-section information (cross section image based upon this) itself with the coordinate system of viewpoint, which is visually confirmed from the front of the occlusal plane. Therefore, the cross-section information desired by the dentist does not always require the cross-section information around the reference axis 206 forming the major axis of the artificial dental root. Specifically, in the case of attempting to incline and implant the artificial dental root, when the inclination is in the mesiodistal direction, desired cross-section information is information around the reference axis 206; however, when the inclination is in the buccolingual direction, the information around the reference axis 206 is not required or will make it difficult to make a determination. Taking these circumstances into consideration, in the embodiments, the reference axis can be inclined both in the tooth alignment direction (mesiodistal direction) and in the buccolingual direction; however, it makes a rule that the plane area 204 where the cross-section information is detected is inclined in the tooth alignment direction along with the reference axis 206 and is rotated around the reference axis only in the case of inclination in the tooth alignment direction, and that even though the reference axis 206 is rotated while the inclined state in the buccolingual direction is maintained on the image display, the plane area 204 is rotated while the non-inclined state in the buccolingual direction is maintained in the case of the inclination in the buccolingual direction. For reference, Fig. 9 is an actual image showing the cross-section information positioned in the plane area 204, and the reference No. 206 represents a reference axis. Fig. 10 is an image showing a positional relationship between the plane area 204 and the maxilla & mandible.

In addition, the three-dimensional imaging information (CT image information) of the maxilla and mandible is overlapped onto the three-dimensional imaging information of models of maxilla and mandible by fitting as described above; however, on the occasion of displaying a cross sectional image based upon the cross-section information positioned in the plane area 204 around the reference axis 206, such as an artificial dental root, it is preferable to display [the image] including the cross-section information of the model. As shown in Fig. 9, the cross section image is displayed with a gray-scale based upon the CT values; however, the shape of soft tissue in the vicinity of tooth alignment (shape of the gums) has a small difference in CT values from the void portion, and it is difficult to visually confirm the outer shape. In the meantime, since the models of the maxilla and mandible (tooth alignment models) are formed with a hollow hard material or thin wall, and the X-absorption rate is also high, if the three-dimensional imaging information of the maxilla and mandible positioned in the plane area 204 and the three-dimensional imaging information of the human body model positioned in the plane area (refer to white broken line in Fig. 9 or the image of gums positioned between the maxillary and mandibular bone images and the tooth alignment image) are extracted, it becomes easy to visually confirm the shape of soft tissue on the same cross sectional image, and the usability for an operator is drastically improved. In addition, in the CT image information as in Fig. 10, on the occasion of executing a process for detecting the cross-section information and detecting an implant position of an artificial dental root; while visually confirming said cross sectional image, the operator fits the artificial dental foot image into the portion missing from a tooth alignment, and at this time, the CT values equivalent to the pre-specified Hounsfield values may be mapped onto the artificial dental root image or the plane area. With this mapping processing, a virtual artificial dental root can be displayed with the gray-scale having the same standard as the CT images of the maxilla and mandible displayed with the gray-scale based upon the CT values, and it is also possible to visually confirm the state of the surface of the artificial dental root and its surrounding human body tissues (cortical bone or cancellous bone) three-dimensionally or two-dimensionally, and the usability for an operator is improved.

As described above, although the embodiments of each device of the present invention have been illustrated and described, the present invention will not be limited to these. For example, in these embodiments, embodiments in the dental field are ordinarily mentioned; however, [the present invention] is also utilizable in other medical fields. Further, the embodiments of the present invention as resolution means of problems in the CT image information have been described; however, it is possible to utilize the present invention for the human body imaging, where the positioning of a patient is difficult, or which includes noise information for other imaging methods, as well.

## Claims

1. A cross-section information detector that extracts three dimensional imaging information of a predetermined plane area including a major axis of a dental implant from three-dimensional imaging information of the maxilla and/or the mandible including a tooth alignment, comprising:
a means that positions the major axis of the dental implant on the three dimensional image of the maxilla and/or the mandible including the tooth alignment;
a means that generates the predetermined plane area including the major axis of the dental implant,
a human body information extraction means that extracts three dimensional imaging information of the maxilla and/or the mandible positioned at said plane area;
wherein the major axis of the dental implant can be inclined in a predetermined direction, and the plane area can be inclined along with the major axis of the dental implant; and
when the major axis of the dental implant is inclined in the predetermined direction, the major axis of the dental implant and the plane area is rotatable while the inclined state is maintained.

## Patentansprüche

1. Querschnittsinformationsdetektor, der dreidimensionale Bildgebungsinformationen einer vorbestimmten ebenen Fläche, einschließlich einer Hauptachse eines Zahnimplantats, aus dreidimensionalen Bildgebungsinformationen des Oberkiefers und/oder Unterkiefers, einschließlich einer Zahnausrichtung, extrahiert, umfassend:
ein Mittel, das die Hauptachse des Zahnimplantats auf dem dreidimensionalen Bild des Oberkiefers und/oder des Unterkiefers, einschließlich der Zahnausrichtung, positioniert;
ein Mittel, das die vorbestimmte ebene Fläche, einschließlich der Hauptachse des Zahnimplantats, erzeugt,
ein Mittel zur Extraktion von Informationen über einen menschlichen Körper, das dreidimensionale Bildgebungsinformationen des Oberkiefers und/oder Unterkiefers, an der ebenen Fläche positioniert, extrahiert;
wobei die Hauptachse des Zahnimplantats in eine vorbestimmte Richtung geneigt sein kann, und die ebene Fläche zusammen mit der Hauptachse des Zahnimplantats geneigt sein kann; und,
wenn die Hauptachse des Zahnimplantats in die vorbestimmte Richtung geneigt ist, die Hauptachse des Zahnimplantats und die ebene Fläche drehbar sind, während der geneigte Zustand aufrechterhalten wird.

## Revendications

1. Détecteur d'informations en coupe transversale, qui extrait des informations d'imagerie tridimensionnelles d'une zone prédéterminée d'un plan incluant un grand axe d'un implant dentaire à partir d'informations d'imagerie tridimensionnelles du maxillaire et/ou de la mandibule, incluant un alignement des dents, comprenant :
un moyen qui positionne le grand axe de l'implant dentaire sur l'image tridimensionnelle du maxillaire et/ou de la mandibule incluant l'alignement des dents ;
un moyen qui génère la zone prédéterminée du plan incluant le grand axe de l'implant dentaire,
un moyen d'extraction d'informations du corps humain qui extrait des informations d'imagerie tridimensionnelle du maxillaire et/ou de la mandibule positionné au niveau de ladite zone du plan ;
le grand axe de l'implant dentaire pouvant être incliné dans une direction prédéterminée et la zone du plan pouvant être inclinée le long du grand axe de l'implant dentaire ; et
quand le grand axe de l'implant dentaire est incliné dans la direction prédéterminée, le grand axe de l'implant dentaire et la zone du plan peuvent tourner tandis que l'état incliné est conservé.
